# EUROPEAN PATENT SPECIFICATION

(11) **EP 0 397 317 B1**
(45) Date of publication and mention of the grant of the patent: **05.10.1994**
(21) Application number: 90303512.9
(22) Date of filing: 02.04.1990
(51) Int. Cl.: C07D 303/24, C08G 59/06

(54) **Diglycidyl ether**
Diglycidyläther
Ethers diglycidiliques

(30) Priority: 21.04.1989 JP 102374/89
(43) Date of publication of application: 14.11.1990
(73) Proprietor: ASAHI DENKA KOGYO KABUSHIKI KAISHA, Arakawa-ku Tokyo 116 (JP)
(72) Inventor: Nambu, Yoko, Yokohama-shi, Kanagawa (JP); Endo, Takeshi, Yokohama-shi, Kanagawa (JP); Abe, Keiji, Arakawa-ku, Tokyo (JP)
(74) Representative: Taylor, Phillip Kenneth

(56) References cited:
- EP-A- 0 155 810
- EP-A- 0 235 990
- EP-A- 0 293 237

## Description

The present invention relates to diglycidyl ethers and a process for preparing the same.

General epoxy resins, e.g., bisphenol A type epoxy resins have been used for a wide variety of applications. These epoxy resins are required to have a further enhanced performance in respect of heat resistance, chemical resistance, flexibility, etc. In the application where particularly high thermal resistance is required, the conventional epoxy resin is unsatisfactory. Even in the case of highly heat-resistant resin, the workability is poor. Therefore, in many cases, the conventional curing process cannot be applied as it is to these resins.

In order to enhance the heat resistance, an attempt has been made on the alteration of a curing agent. However, in this case as well, the conventional cure molding process should be unfavorably modified.

Accordingly, it has been desired to develop a highly heat-resistant epoxy resin which is excellent in the workability and can be handled under the same cure molding condition as that used in general epoxy resins.

The present inventors have made extensive and intensive studies with a view to solving the above-described problem and, as a result, have found that diglycidyl ethers having an aromatic amide structure have very high heat resistance and provide very excellent workability.

Specifically, the diglycidyl ethers of the present invention are characterized by having the following general formula (1): 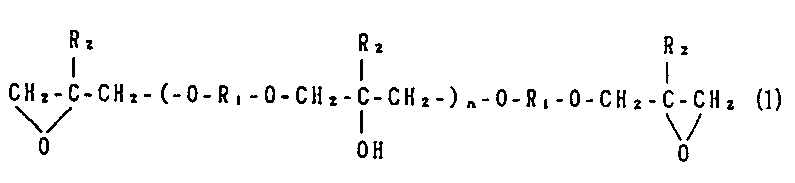
wherein R₁ is 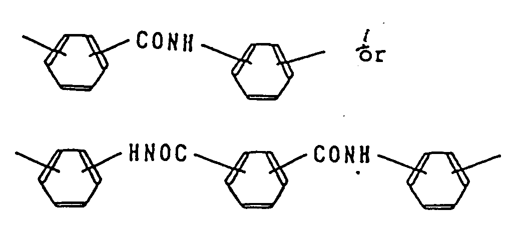
R₂ is a hydrogen atom or a methyl group and n is a number of 0 to 3.

Further, the diglycidyl ethers of the present invention may be prepared by a process characterized by reaction of a phenolic compound represented by the general formula (2):

HO-R₁-OH (2)

wherein R₁ is as defined above,
with an epihalohydrin represented by the general formula (3): 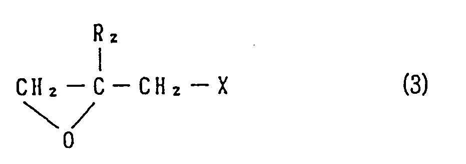
wherein R₂ is as defined above and X is a halogen atom.

The phenolic compound represented by the general formula (2) may be prepared by reacting terephthaloyl dichloride or isophthaloyl dichloride with p-aminophenol or m-aminophenol in an amide solvent.

The phenolic compound represented by the general formula (2) according to the present invention may be prepared also by subjecting the product of a reaction of p-acetoxybenzoyl chloride or m-acetoxybenzoyl chloride with p-aminophenol or m-aminophenol in an aqueous alcohol solution to treatment with an alkali carbonate.

The epihalohydrin represented by the general formula (3) according to the present invention may be a general epihalohydrin, and examples thereof include epichlorohydrin, β-methylepichlorohydrin, epibromohydrin and β-methylepibromohydrin. Epichlorohydrin is preferably used.

The reaction of the phenolic compound of the present invention represented by the general formula (2) with the epihalohydrin of the present invention represented by the general formula (3) is preferably conducted in the presence of an alkali hydroxide in an amide solvent. In this case, the above-described epihalohydrin is used in an amount of 2 to 100 times by mole, preferably 10 to 50 times by mole that of the phenolic compound.

Examples of the alkali hydroxide which may be used in the present invention include sodium hydroxide, potassium hydroxide, barium hydroxide and calcium hydroxide, among which sodium hydroxide is preferably used.

The alkali hydroxide is used in a slightly excess based on the hydroxyl group of the phenolic compound represented by the general formula (2).

The reaction temperature is 40 to 150°C, preferably 60 to 120°C. The reaction time is 0.5 to 10 h, preferably 1 to 5 h.

The solvent used in the reaction is preferably an amide solvent, such as N,N-dimethylacetamide N,N-dimethylformamide and N-methylpyrrolidone. It is preferred to use the solvent in the minimal amount necessary for the phenolic compound represented by the above-described general formula (2) to be dissolved therein.

The diglycidyl ether represented by the general formula (1) may be used alone or in the form of a mixture thereof with other known epoxy resin. Examples of the above-described epoxy resin include known diglycidyl ethers, such as bisphenol A diglycidyl ether, cyclohexene oxides, and polyglycidyl amines of polyvalent anilines.

The conventional epoxy resin curing agents known in the art may be used for the purpose of curing the diglycidyl ether represented by the general formula (1). Examples of the curing agent include latent curing agents such as amines, acid anhydrides, novolak resins, and dicyandiamides.

The above-described curing agents may be used in an amount of 0.5 to 200 parts by weight, preferably 5 to 50 parts by weight based on 100 parts by weight of the diglycidyl ether of the present invention represented by the general formula (1).

Further, the diglycidyl ethers of the present invention represented by the general formula (1) are used after being mixed with various additives, fillers, etc. which are usually used in the art.

The diglycidyl ether of the present invention represented by the general formula (1) can provide a cured product having excellent heat resistance through curing with a general curing agent and curing system and can be used for a wide variety of applications as resins for laminates as well as for various molding materials, casting, impregnation and coating.

The present invention will now be described in more detail by way of the following Examples.

### Example 1

52 g of p-aminophenol was dissolved in 620 g of N-methylpyrrolidone, and a solution of 21 g of terephthaloyl dichloride in methylene dichloride was dropwise added thereto over a period of 1 hr at room temperature. The mixture was allowed to react at room temperature for 3.5 hr and then at 50°c for 2 hr. The reaction mixture was poured into water. The resultant precipitate was washed with 1 N HCl and water and dried to obtain 30 g of a pale yellow powder. Analysis by ¹H-NMR (dmso-d₆) gave peaks respectively at the following positions:
δ (ppm)
10.08 (s, 2H, NH) 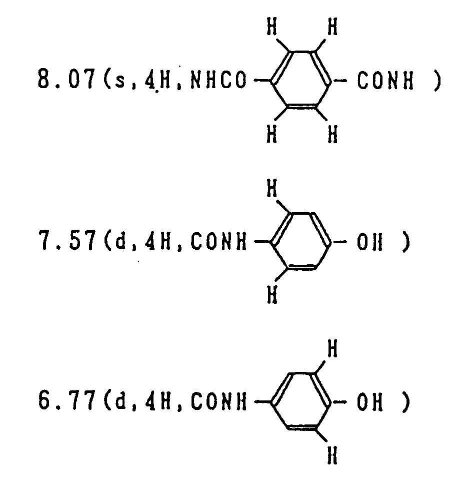
Analysis by IR (KBr, cm⁻¹) gave peaks respectively at the following positions: 3373, 3321 (OH, NH), 1639 (CONH), 1539 (CONH), 1250 (CONH), 829 (Ph). Therefore, this product has the following structure. 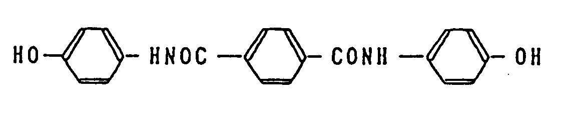
Melting point > 300°C
The obtained powder was dissolved in 202 g of epichlorohydrin and 820 g of N-methylpyrrolidone, and 34.5 g of a 20 % aqueous sodium hydroxide solution was added thereto. The mixture was allowed to react at 70°C for 1.5 h and at 80°c for 3 h. Then water and epichlorohydrin were distilled off in vacuo from the reaction mixture. The residue was poured into water, and the formed precipitates were collected by filtration. The precipitates were dissolved in N,N-dimethylformamide, and diatomaceous earth was added thereto. The mixture was subjected to filtration, and N,N-dimethylformamide was distilled off, thereby preparing 38 g of diglycidyl ether of the present invention in the form of a pale brown solid.

Analysis of this product by ¹H-NMR (dmso-d₆) gave the following peaks in an epoxy methylene proton/benzene ring proton ratio of 88 % of the stoichiometric value:
δ(ppm)
10.18 (s, 2H, NH) 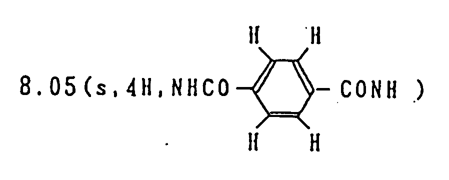 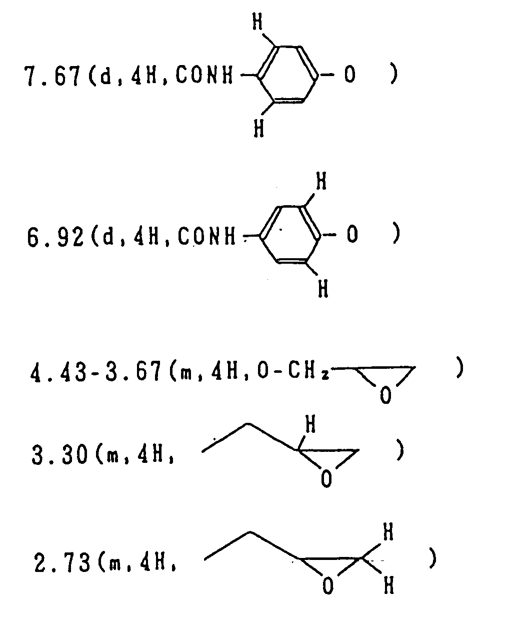
Analysis by IR (KBr, cm⁻¹) gave peaks respectively at the following positions: 3319 (NH), 1639 (CONH), 910 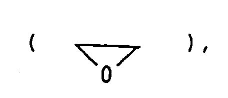
and analysis by ¹³C-NMR (dmso-d₆) gave peaks respectively at the following positions: δ (ppm) 164.4, 154.7, 137.4, 132.4, 127.5, 122.1, 114.6, 69.1, 49.7, 43.7.

Therefore, this diglycidyl ether is represented by the general formula (1) wherein
R₁ is 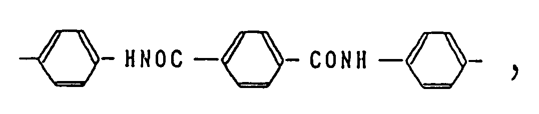
R₂ is H and n is 1.

### Example 2

72 g of m-aminophenol was dissolved in 460 g of N-methylpyrrolidone, and a solution of 32 g of isophthaloyl dichloride in tetrahydrofuran was dropwise added thereto over a period of 20 min. The temperature was gradually raised up to 80°C over a period of 5 h, and the reaction was conducted at 80°C for 3 h as it was. The reaction mixture was allowed to stand for cooling and poured into water. The formed precipitates were collected by filtration, washed with water, and dried to prepare 53 g of an ashy white powder. Analysis by ¹H-NMR (dmso-d₆) gave peaks respectively at the following positions:
δ(ppm)
10.13 (s, 2H, NH)
9.28 (s, 2H, OH)
8.43-6.50 (m, 12H, Ph).
Analysis by IR (KBr, cm⁻¹) gave peaks respectively at the following positions: 3383, 3310, 3242 (OH, NH), 1631, 1547, 1265 (CONH), 775 (Ph). Therefore, the product has the following structure: 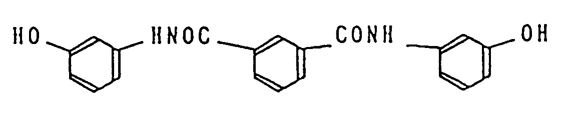
Melting point: 257 to 259°C
The obtained powder was dissolved in 352 g of epichlorohydrin, 1200 g of isopropanol and 400 g of N-methylpyrrolidone, and 60.8 g of a 20 % aqueous sodium hydroxide solution was added thereto. The mixture was allowed to react at 60°C for 4 h and poured into water. The formed precipitates were collected by filtration and dried to prepare 66 g of diglycidyl ether of the present invention in the form of a white solid.

Analysis of this product by ¹H-NMR (dmso-d₆) gave the following peaks in an epoxy methylene proton/benzene ring proton ratio of 91 % of the stoichiometric value:
δ(ppm)
10.42 (s, 2H, NH)
8.57-6.73 (m, 12H, Ph) 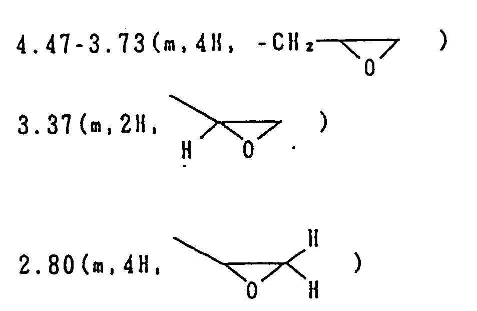
Analysis by IR (KBr, cm⁻¹) gave peaks respectively at the following positions: 3450 (NH), 1645, 1255 (CONH), 910 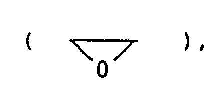
and analysis by ¹³C-NMR (dmso-d₆) gave peaks respectively at the following positions: δ (ppm) 165.0, 158.3, 140.2, 135.1, 130.6, 129.4, 128.5, 126.9, 112.8, 109.7, 106.6, 68.8, 49.6 and 43.7. The melting point was 170 to 176°C.

Therefore, the diglycidyl ether is represented by the general formula (1) wherein
R₁ is 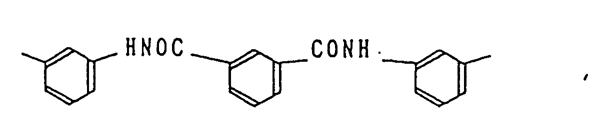
R₂ is H and n is 1.

### Example 3

53 g of p-aminophenol was dissolved in 375 g of N,N-dimethylbenzoyl chloride, and 44 g of p-acetoxybenzoyl chloride was dropwise added thereto over a period of 1.5 h. The temperature was gradually raised to 113°C over a period of 3 h. The reaction mixture was allowed to stand for cooling and poured into water, and the formed precipitates were collected by filtration and washed with 1 N HCl and water and dried to prepare 44 g of a white powder. Analysis by IR (KBr, cm⁻¹) of this product gave peaks respectively at the following positions: 3445, 3340 (OH, NH), 1751 (COO) and 1651 (CONH).

The white powder was dissolved in 4000 g of methanol and 2400 g of water, and 5 g of sodium carbonate was added thereto. The mixture was allowed to react for 2 hr. The reaction mixture was neutralized with 4 N HCl, and it was confirmed that the liquid was neutral when methanol was distilled off in vacuo. Water was distilled off in vacuo, and the residue was dried. The residue was purified by silica gel column chromatography to prepare 35 g of a diol. Analysis by ¹H-NMR (CD₃OD) gave peaks respectively at the following positions: δ (ppm) 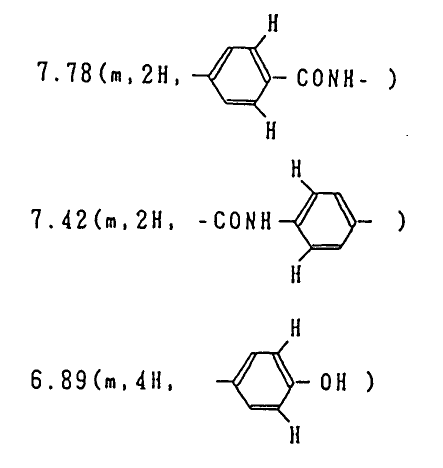
4.78 (br, 3H, OH, NH)
Analysis by IR (KBr, cm⁻¹) gave peaks respectively at the following positions: 3320 (OH), 1647, and 1246 (CONH). Therefore, the product has the following structure: 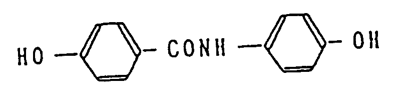
Melting point: 258-262°C (decomposed)
The diol thus prepared was dissolved in 393 g of epichlorohydrin and 350 g of N-methylpyrrolidone, and 73.9 g of a 16.6 % aqueous sodium hydroxide solution was dropwise added thereto. The mixture was allowed to react at 75°C for 1.5 h.

Water and epichlorohydrin were distilled off in vacuo from the reaction mixture, and insolubles were filtered off. The filtrate was poured into water to form precipitates. The precipitates were collected by filtration and dried to prepare 55 g of diglycidyl ether of the present invention in the form of a white solid.

Analysis of this product by ¹H-NMR (dmso-d₆) gave peaks respectively at the following positions in an epoxy methylene proton/benzene ring proton ratio of 91 % of the stoichiometric value:
δ(ppm)
9.90(s, 1H, NH) 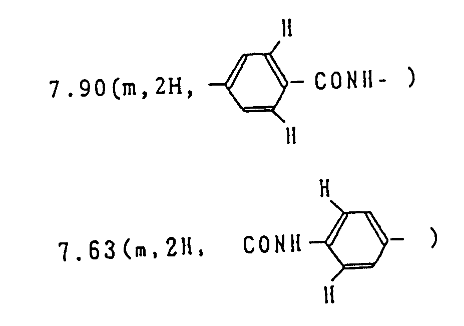 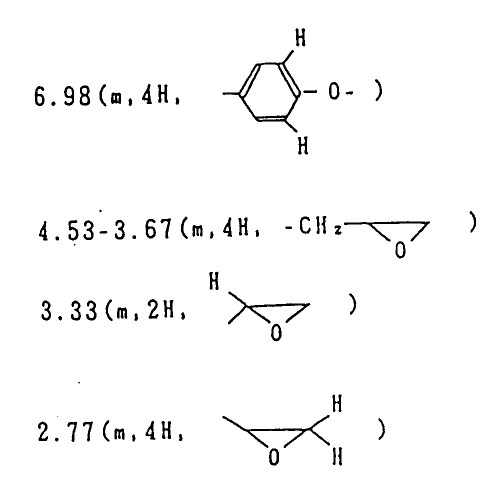
Analysis by IR (KBr, cm⁻¹) gave peaks respectively at the following positions: 3310 (NH), 1645, 1255 (CONH), 915 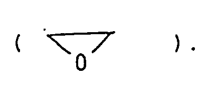
The melting point was 169 to 173°C.

Therefore, the diglycidyl ether is represented by the general formula (1) wherein
R₁ is 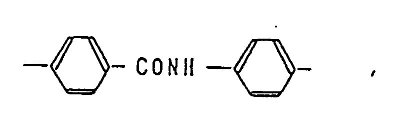
R₂ is H and n is 1.

### Example 4

56 g of m-aminophenol was dissolved in 300 g of N,N-dimethylacetamide, and 46 g of p-acetoxybenzoyl chloride was dropwise added thereto over a period of 45 min. Thereafter, the temperature was gradually raised to 100°C over a period of 2 h. After the reaction mixture was allowed to stand for cooling, 400 g of water and 400 g of 1 N HCl were added thereto. The mixture was extracted with ethyl acetate. The resultant organic phase was concentrated and dried to obtain 64 g of a white solid. Analysis by IR (KBr, cm⁻¹) gave peaks respectively at the following positions: 3410 (OH), 3364 (NH), 1751 (COO) and 1655 (CONH).

This white solid was dissolved in 3850 g of methanol and 1500 g of water, and 13 g of sodium carbonate was added thereto. The mixture was allowed to react for 1.5 hr. The reaction mixture was neutralized with 6 N HCl, concentrated and dried. The residue was purified by silica gel column chromatography and recrystallized from chloroform-ethyl acetate to obtain 24 g of a diol in the form of a white crystal. The melting point was 229 to 230°C. Analysis of this product by ¹H-NMR (CD₃OD) gave peaks respectively at the following positions: δ (ppm) 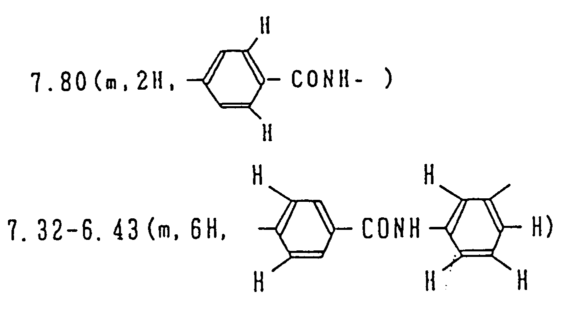
4.83(s, 3H, NH, OH)
and analysis by IR (KBr, cm⁻¹) gave peaks respectively at the following positions: 3325, 3260 (NH, OH) and 1630 (CONH). Therefore, the product has the following structure. 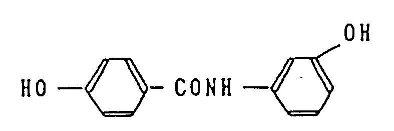
The white crystal thus prepared was dissolved in 196 g of epichlorohydrin and 67 g of N-methylpyrrolidone, and 41.7 g of a 20 % aqueous sodium hydroxide solution was dropwise added thereto. The mixture was allowed to react at 65°C for 2 h.

Water and epichlorohydrin were distilled off in vacuo from the reaction mixture. When water was again added to the reaction mixture, there occurred solidification of the reaction mixture. The solid was recovered and dried to obtain 33 g of a pale yellow solid.

Analysis of this product by ¹H-NMR (acetone-d₆) gave peaks respectively at the following positions in an epoxy methylene proton/benzene ring proton ratio of 89 % of the stoichiometric value: δ(ppm)
9.43(s, 1H, NH) 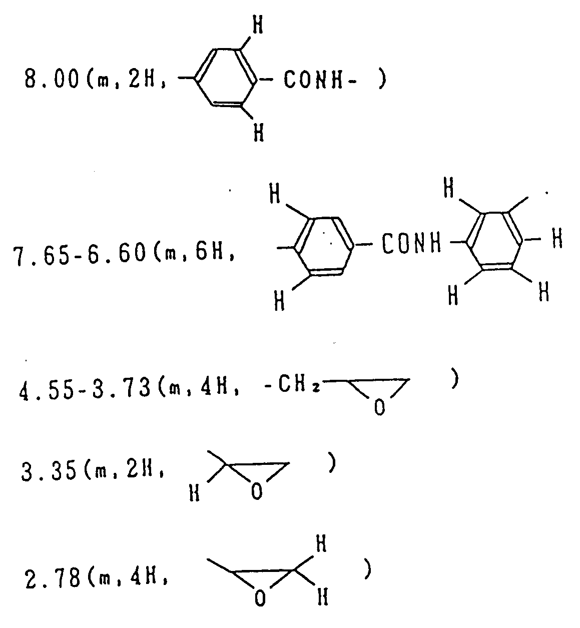
Analysis by IR (KBr, cm⁻¹) gave peaks respectively at the following positions: 3330 (NH), 1660, 1260 (CONH), 915

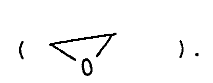

Therefore, this diglycidyl ether is represented by the general formula (1) wherein
R₁ is 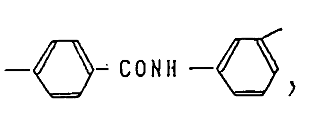
R₂ is H and n is 1.

### Example 5

A cured product prepared by adding 18.9 g of diaminodiphenylmethane to 100 g of the diglycidyl ether prepared in Example 1 and curing the mixture at 230°C for 6 h exhibited a Tg value of 226°C as determined by DSC.

A cured product prepared by adding 8.4 g of dicyandiamide and 0.21 g of 2-ethyl-4-methylimidazole to 50 g of the diglycidyl ether prepared in Example 1 and 50 g of a bisphenol A epoxy resin (EP-4100S having an epoxy equivalent of 183; a product of Asahi Denka Kogyo K.K.) and curing the mixture at 180°C for 4 h exhibited a Tg value of 145°C as determined by DSC. A cured product prepared by adding 23.0 g of diaminodiphenylmethane to the diglycidyl ether and curing the mixture at 220°C for 4 h exhibited a Tg value of 137°C.

### Example 6

A cured product prepared by adding 7.1 g of dicyandiamide and 0.18 g of 2-ethyl-4-methylimidazole to 100 g of the diglycidyl ether prepared in Example 2 and curing the mixture at 220°C for 4 h exhibited a Tg value of 166°C as determined by DSC, and a cured product prepared by adding 19.6 g of diaminodiphenylmethane to the diglycidyl ether and curing the mixture at 180°C for 4 h exhibited a Tg value of 147°C.

A cured product prepared by adding 8.5 g of dicyandiamide and 0.21 g of 2-ethyl-4-methylimidazole to 50 g of the diglycidyl ether prepared in Example 2 and 50 g of a bisphenol A epoxy resin (EP-4100S having an epoxy equivalent of 183; a product of Asahi Denka Kogyo K.K.) and curing the mixture at 180°C for 4 h exhibited a Tg value of 161°C as determined by DSC, and a cured product prepared by adding 23.3 g of diaminodiphenylmethane to the diglycidyl ether and curing the mixture at 220°C for 4 h exhibited a Tg value of 138°C.

### Example 7

A cured product prepared by adding 9.6 g of dicyandiamide and 0.24 g of 2-ethyl-4-methylimidazole to 100 g of the diglycidyl ether prepared in Example 3 and curing the mixture at 180°C for 4 h exhibited a Tg value of 162°C as determined by DSC, and a cured product prepared by adding 26.4 g of diaminodiphenylmethane to the diglycidyl ether and curing the mixture at 180°C for 4 h exhibited a Tg value of 152°C.

A cured product prepared by adding 9.7 g of dicyandiamide and 0.24 g of 2-ethyl-4-methylimidazole to 50 g of the diglycidyl ether prepared in Example 3 and 50 g of a bisphenol A epoxy resin (EP-4100S having an epoxy equivalent of 183; a product of Asahi Denka Kogyo K.K.) and curing the mixture at 180°C for 4 hr exhibited a Tg value of 156°C as determined by DSC, and a cured product prepared by adding 26.7 g of diaminodiphenylmethane to the diglycidyl ether and curing the mixture at 180°C for 4 h exhibited a Tg value of 145°C.

### Example 8

A cured product prepared by adding 9.4 g of dicyandiamide and 0.23 g of 2-ethyl-4-methylimidazole to 100 g of the diglycidyl ether prepared in Example 4 and curing the mixture at 200°C for 4 h exhibited a Tg value of 127°C as determined by DSC, and a cured product prepared by adding 25.8 g of diaminodiphenylmethane to the diglycidyl ether and curing the mixture at 200°C for 4 h exhibited a Tg value of 133°C.

A cured product prepared by adding 9.6 g of dicyandiamide and 0.24 g of 2-ethyl-4-methylimidazole to 50 g of the diglycidyl ether prepared in Example 4 and 50 g of a bisphenol A epoxy resin (EP-4100S having an epoxy equivalent of 183; a product of Asahi Denka Kogyo K.K.) and curing the mixture at 100°C for 4 h exhibited a Tg value of 137°C as determined by DSC, and a cured product prepared by adding 26.7 g of diaminodiphenylmethane to the diglycidyl ether and curing the mixture at 180°C for 4 h exhibited a Tg value of 146°C.

### Comparative Example 1

A cured product prepared by adding 9.8 g of dicyandiamide and 0.25 g of 2-ethyl-4-methylimidazole to 100 g of a bisphenol A epoxy resin (EP-4100S having an epoxy equivalent of 183; a product of Asahi Denka Kogyo K.K.) and curing the mixture at 180°C for 3 h exhibited a Tg value of 138°C as determined by DSC, and a cured product prepared by adding 27.1 g of diaminodiphenylmethane to the epoxy resin and curing the mixture at 160°C for 4 h exhibited a Tg value of 130°C.

## Claims

1. A diglycidyl ether represented by the following general formula (1): 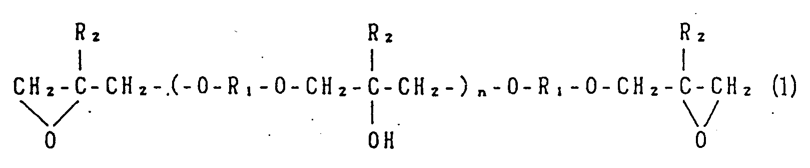 wherein R₁ is 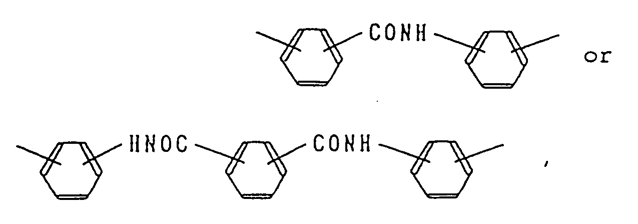 R₂ is a hydrogen atom or a methyl group and n is a number of 0 to 3.

2. A process for preparing a diglycidyl ether according to claim 1, characterized by reacting a phenolic compound represented by the general formula (2):
HO-R₁-OH (2)
wherein R₁ is as defined in claim 1
with an epihalohydrin represented by the general formula (3): 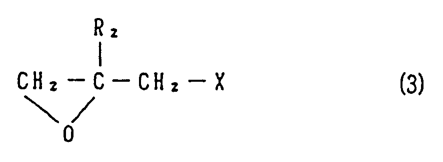 wherein R₂ is as defined in claim 1 and X is a halogen atom.

## Patentansprüche

1. Diglycidylether, das durch die folgende allgemeine Formel (1) ausgedrückt wird: 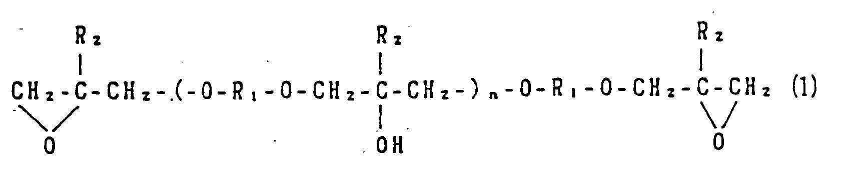 wobei R₁ 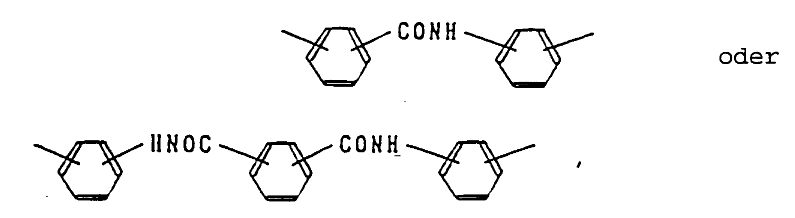 R₂ ein Wasserstoffatom oder eine Methylgruppe und n eine Zahl zwischen 0 und 3 ist.

2. Verfahren zur Zubereitung eines Diglycidylethers nach Anspruch 1, dadurch gekennzeichnet, daß eine Phenolverbindung, die durch die allgemeine Formel (2)
HO-R₁-OH (2)
ausgedrückt wird, wobei R₁ der Definition in Anspruch 1 entspricht, mit einem durch die allgemeine Formel (3) 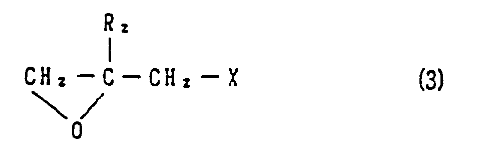 ausgedrückten Epihalohydrin reagiert wird, wobei R₂ der Definition in Anspruch 1 entspricht und X ein Halogenatom ist.

## Revendications

1. Ether de diglycidyle représenté par la formule générale suivante (1): 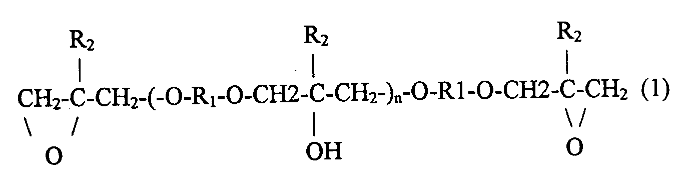 où R1 est 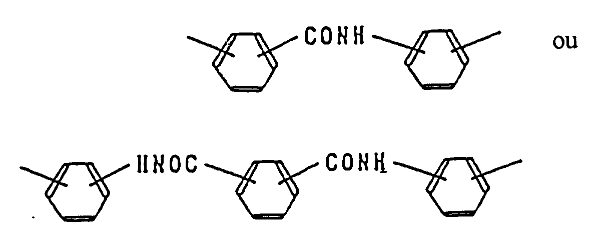 R₂ est un atome d'hydrogène ou un groupe méthyle et n est un nombrre de 0 à 3.

2. Procédé de préparation d'un éther de diglycidyle selon la revendication 1, caractérisé par la réaction d'un composé phénolique représenté par la formule générale (2):
HO-R₁-OH (2)
où R₁ est tel que défini dans la revendication 1, avec une épihalohydrine représentée par la formule générale (3): 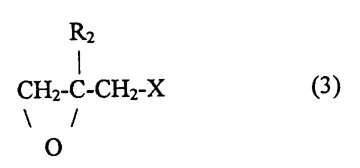 où R₂ est tel que défini dans la revendication 1 et X est un atome d'halogène.
